# EUROPEAN PATENT APPLICATION

(11) **EP 1 190 696 A2**
(43) Date of publication of application: **27.03.2002**
(21) Application number: 01122516.6
(22) Date of filing: 21.09.2001
(51) Int. Cl.: A61K 7/06

(54) **Hair cosmetic compositions and use thereof**

(30) Priority: 21.09.2000 JP 2000286644
(71) Applicant: Kao Corporation, Tokyo 103-8210 (JP)
(72) Inventor: Tajima, Sachiko, Sumida-ku, Tokyo 131-8501 (JP); Shibue, Fumio, Sumida-ku, Tokyo 131-8501 (JP); Kaneko, Kenichi, Sumida-ku, Tokyo 131-8501 (JP)
(74) Representative: HOFFMANN - EITLE

(57) **Abstract**

A hair cosmetic formulation, comprising:
(A) a fragrance ingredient comprising cis-3-hexenol; and
(B) at least one ingredient selected from the group consisting of ammonia, monoethanolamine, and an aromatic alcohol penetration promoter. This formulation may be used as a hair coloring formulation.

## Description

### BACKGROUND OF THE INVENTION

### Field of the Invention

The present invention relates to hair cosmetic compositions, which have a fragrance with an unpleasant smell masked although such an unpleasant smell would otherwise be given off from ammonia, monoethanolamine, or a penetration promoter of the aromatic alcohol type.

### Background of the Invention

In hair dye or coloring formulations (hereinafter collectively called "hair coloring formulations"), alkaline agents such as ammonia and monoethanolamine are frequently incorporated therein. The ammonia and amine smells, which these compositions inherently have, have remained unsolved as a serious problem for both those applying the hair coloring formulations and those treated with the formulations. Thus, there has been a long-standing desire for the development of a method for masking ammonia and amine odors inherent to these formulations. In many hair cosmetic compositions with such hair coloring formulations incorporated therein, on the other hand, a penetration promoter of the aromatic alcohol type is added to promote the penetration of ingredients which act on hair. However, this penetration promoter also imparts a solvent smell to the compositions. These factors have led to a significant need for the development of a method by which the odor of such compositions can be masked.

### SUMMARY OF THE INVENTION

Accordingly, one object of the present invention is to provide a hair coloring formulation which effectively masks the odor(s) inherent to solvent, ammonia and amine containing hair coloring formulations.

Briefly, this object and other objects of the present invention as hereinafter will become more readily apparent can be attained by a hair cosmetic formulation, which comprises: (A) a fragrance ingredient comprising cis-3-hexenol, and (B) at least one ingredient selected from the group consisting of ammonia, monoethanolamine, and an aromatic alcohol penetration promoter.

### DETAILED DESCRIPTION OF THE INVENTION

With a view to ascertaining how to mask the unpleasant smells of hair cosmetic compositions containing ammonia, monoethanolamine and aromatic alcohols, an investigation has been conducted into the effectiveness of a variety of fragrant materials in masking undesirable odors. As a result, it has been found that addition of cis-3-hexenol, which is called "leaf alcohol" and which has not been considered to be readily useful in formulations as a fragrance because of its grassy smell hair, in an amount greater than its usual amount results in a hair cosmetic composition which has a very desirable fragrance.

The hair cosmetic composition of the present invention has a desirable fragrance in which the odor of ammonia, amine or solvent is effectively masked. The composition also has good stability.

The fragrance ingredient (A) of the present invention contains cis-3-hexenol. As mentioned above, cis 3-hexenol is known as "leaf alcohol" and is a fragrance which is useful for imparting a grassy smell to a formulation containing it. It was, however, not known that this compound is able to mask the odors of ammonia, amines and solvent which may be in a composition. To achieve the effective masking and fragrance effect of the invention, it is preferred to add cis-3-hexenol to a composition in an amount of 0.1 to 50 wt.% (hereinafter indicated simply by "%"), especially from 1 to 30% in ingredient (A).

The ingredient (A) may preferably contain, in addition to cis-3-hexenol, one or more substances selected from the group consisting of cis-3-hexenol esters such as cis-3-hexenyl acetate, cis-3-hexenyl formate, cis-3-hexenyl propionate and cis-3-hexenyl salicylate, alcohol C-6, trans-2-hexenol, dimethol (2,6-dimethyl-2-heptanol), dihydromyrcenol (2,6- dimethyl-7-octen-2-ol), citronellol (3,7-dimethyl-6-octen-1-ol), geraniol (3,7-dimethyl-cis-2,6-octadien-1-ol), linalool (3,7-dimethyl-1,6-octadien-3-ol), Magnol™, Sandalmysore Core™ [2-methyl-5-(2,2,3-trimethyl-3-cyclopenten-1-yl)-4-penten-2-ol, product of Kao S.A.], eugenol [2-methoxy-4-(1-propenyl)-phenol], p-cresol, Herbac™ (3,3-dimethylcyclohexyl methyl ketone), Koavone™ (acetyl diisoamylene), -methyl ionone [5-(2,6,6-trimethyl-2-cyclohexen-l-yl)-3-methyl-3-buten-2-one], 1-menthone (o-menthan-3-one), Liffarome™ (cis-3-hexenyl methyl carbonate), Manzanate™ (ethyl 2-methylpentanoate, product of Quest Int'1 U.K. Ltd.), Fruitate™ [ethyl tricylo[5.2.1.0^{2,6}]decan-2-ylcarboxylate], o-t-B.C.H.A.(o-t-butylcyclohexyl acetate), p-cresyl acetate, 1,8-cineole, Anethole™, estragol (methyl chavicol), rose oxide [4-methyl-2-(2-methyl-1-propenyl)-tetrahydropyrate] and limonene [p-mentha-1,4(8)-diene]. The ingredient (A) may further contain a solvent as a diluent. Suitable such solvents include dipropylene glycol, triethyl citrate and ethanol. From the standpoint of the emission of a preferred fragrance, the hair cosmetic composition of the present invention can preferably contain ingredient (A) in an amount ranging from 0.1 to 1.0%, especially from 0.3 to 0.8%, based on the weight of the composition.

Suitable ingredients (B)of the present hair cosmetic composition include ammonia, which is commonly used as an alkaline agent in oxidation hair colors and hair bleaches. The hair cosmetic composition preferably contains ammonia in an amount of 0 to 3%, especially from 0 to 1%, when the hair cosmetic composition is in a form ready for application onto hair (in other words, in a mixed form). The penetration promoter of the aromatic alcohol type, on the other hand, is used to promote penetration of an ingredient which acts on the interior of hair, such as a penetration promoter for a dye in a hair color, a penetration promoter for an active ingredient in a shampoo or conditioner, or a penetration promoter for an active ingredient in a hair fixative, and is preferably present in an amount ranging from 0 to 40%, especially 5 to 25% in the cosmetic hair composition. Here, it should be noted that the amounts of the above-described three ingredients (B) are not all 0% at the same time.

Suitable examples of the penetration promoter of the aromatic alcohol type of the invention include aromatic alcohols represented by the following formula (1): 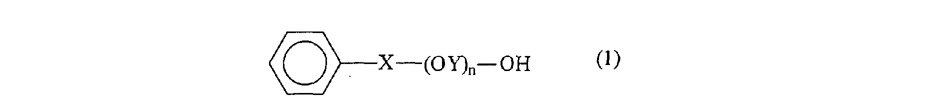 wherein n stands for 0 or 1. When n=0, X is a linear or branched alkylene, alkenylene or alkylenoxy group having 1 to 6 carbon atoms, with the proviso that the oxygen atom of the alkylenoxy group is bonded to the benzene ring and, when n=1, X and Y each independently represent a linear or branched alkylene group having 1 to 6 carbon atoms.

In formula (1), when n=0, X is preferably a linear or branched alkylene or alkylenoxy group having 1 to 4 carbon atoms and, when n=1, X and Y is preferably a methylene group or a linear alkylene group having 2 to 6 carbon atoms. Specific examples of the aromatic alcohols include benzyl alcohol, phenylethyl alcohol, phenoxyethanol, phenoxyisopropanol, -methylbenzyl alcohol, , -dimethylbenzyl alcohol, -propylbenzyl alcohol, 2-benzyloxyethanol, and 3-benzyloxybutanol, with benzyl alcohol and 2-benzyloxyethanol being particularly preferred.

Illustrative hair cosmetic compositions of the present invention include oxidation hair coloring formulations, hair bleaching formulations, acid-dye-based hair coloring formulations and basic-dye-based hair coloring formulations, all of whose ammonia odor, amine odor and/or solvent odor is as described above. Of these, oxidation coloring formulations are particularly preferred. In the case of an oxidation coloring, an oxidation dye intermediate, for example, a color-developing substance and a coupling agent are added. Suitable examples of the color-developing substance include p-phenylenediamines, 2,5-diaminopyridines, p-aminophenols, o-aminophenols, o-phenylenediamines, and 4,5-aminopyrazoles. Suitable examples of the coupling agent, on the other hand, include various m-phenylenediamines, m-aminophenols, m-hydroxybenzenes, hydroxyindoles, naphthols, and phenols. In addition, a direct dye or the like can also be incorporated in the composition.

When an oxidation dye intermediate is incorporated in a hair cosmetic composition of the present invention, oxidative coupling is induced with oxygen in the air, an enzyme or the like to color the hair or the like. To induce this oxidative coupling, addition of a chemical oxidizing agent is more preferred. Illustrative of the chemical oxidizing agents include hydrogen peroxide, hydrogen peroxide solution, e. g., 35%, urea peroxide, alkali metal bromates, and alkali metal peracid salts, such as perbromates, persulfates or perborates, with hydrogen peroxide being particularly preferred.

In the hair cosmetic composition of the present invention, viscosity/gel strength modifiers, oils and fats, waxes, hydrocarbons, polyhydric alcohols, amides, silicone derivatives, cationic surfactants, anionic surfactants, amphoteric surfactants, nonionic surfactants, nonionic high-molecular substances, cationic high-molecular substances, anionic high-molecular substances, amphoteric high-molecular substances, protein derivatives and amino acids, preservatives, chelating agents, stabilizers, oxidation inhibitors, plant extracts, crude drug extracts, vitamins, color additives, fragrances, pigments, ultraviolet absorbers and the like can be incorporated therein as additives.

The pH of the hair cosmetic composition of the present invention preferably ranges from 8 to 12, notably from 9 to 11 when it is an oxidation hair coloring formulation.

### EXAMPLES

### Example 1

As fragrances in the following hair color cream formula, hair color lotion formula and hair manicure formula, the below-described fragrance formula (A) and fragrance formula (B) was added.

Having now generally described this invention, a further understanding can be obtained by reference to certain specific examples which are provided herein for purposes of illustration only and are not intended to be limiting unless otherwise specified.

| 1) Hair color cream formula (Pack 1) | (%) |
|---|---|
| Aqueous ammonia (28%) | 1.0 |
| Ammonium bicarbonate | 1.3 |
| Ferrous sulfate | 20 ppm |
| Tetrasodium ethylenediaminetetraacetate | 0.2 |
| Monoethanolamine | 3.0 |
| Monoethanolamine HCl solution (60%) | 1.2 |
| Potassium carbonate | 2.0 |
| Toluene-2,5-diamine | 1.5 |
| Resorcin | 0.6 |
| m-Aminophenol | 0.3 |
| Oleic acid | 10.0 |
| Diethanol oleic acid amide | 8.0 |
| POE(20) octyldodecyl ether | 10.0 |
| Ethanol | 15.0 |
| Propylene glycol | 10.0 |
| Sodium sulfite | 0.5 |
| Ascorbic acid | 0.5 |
| Fragrance | 0.5 |
| Potassium hydrogencarbonate | q.s. to pH 11.0 |
| Water | Balance |

| 2) Liquid formulation for hair color (1 part agent) | (%) |
|---|---|
| Sodium lauroylglutaminate | 5.0 |
| Sodium lauroylsulfate | 5.0 |
| Sodium alkanesulfonate | 3.0 |
| Polyoxyethylene (3) tridecyl ether | 39.0 |
| 2-Benzyloxyethanol | 25.0 |
| Monoethanolamine | 4.0 |
| Aqueous ammonia (28%) | 3.5 |
| Anhydrous sodium sulfite | 0.4 |
| Toluene-2,5-diamine | 1.0 |
| m-Aminophenol | 1.0 |
| Merquat 550 (product of Goodrish) | 4.5 |
| Perfume | 0.5 |
| Water | Balance |

| * 2-part agent for hair color | (%) |
|---|---|
| Sodium polyoxyethylene (2) lauryl ether sulfate | 15.0 |
| Polyoxyethylene (9) lauryl ether | 5.0 |
| 35% Aqueous hydrogen peroxide | 17.0 |
| 75% Aqueous solution of phosphoric acid | 0.3 |
| Water | Balance |

| 3) Hair manicure formula | (%) |
|---|---|
| Ethanol | 10.0 |
| 1,3-Butylene glycol | 10.0 |
| Benzyl alcohol | 5.0 |
| Citric acid | 5.0 |
| Isostearyl pentaerythryl glyceryl ether | 0.1 |
| Polyether-modified silicone | 0.5 |
| Xanthan gum | 1.5 |
| Black No. 401 (CI 20470) | 0.03 |
| Orange No. 205 (CI 15510;D&C Orange No.4) | 0.06 |
| Fragrance | 0.5 |
| Water | Balance |

| Fragrance formula (A) | (%) |
|---|---|
| cis-3-Hexenol | 20 |
| Sandalmysore Core™ | 5 |
| -Methyl ionone | 5 |
| Linalool | 10 |
| Eugenol | 10 |
| Estragol | 2 |
| Fruitate™ | 5 |
| Limonene | 10 |
| Hexyl salicylate | 5 |
| Floral base | 20 |
| Fruit base | 5 |
| Dipropylene glycol | 3 |
| TOTAL | 100 |

| Fragrance formula (B) | (%) |
|---|---|
| Hexyl salicylate | 5 |
| Floral base | 20 |
| Fruit base | 5 |
| Citral | 5 |
| Hexylcinnamic aldehyde | 20 |
| Lyral™ | 10 |
| Lilial™ | 10 |
| Helional | 5 |
| Methylnonyl acetaldehyde | 5 |
| Benzyl salicylate | 5 |
| Dipropylene glycol | 5 |
| TOTAL | 100 |

The hair color cream formula, the hair color lotion formula and the hair manicure formula were organoleptically ranked in smell in accordance with the following 5-stage ranking system by a panel consisting of 5 experts. The results are shown as averages of scores in Table 1.
5: Preferred fragrance
4: No irritating smell
3: Noticeable ammonia smell/solvent smell
2: Strong initation of ammonia smell/solvent smell
1: Very strong irritation of ammonia smell/solvent smell Table 1

**TABLE 1**

| | Examples Fragrance Formula (A) | Comparative Examples Fragrance Formula (B) |
|---|---|---|
| 1) Hair coloring cream formula | 4.8 | 2.4 |
| 2) Hair coloring lotion formula | 4.6 | 2.2 |
| 3) Hair manicure formula | 4.4 | 2.0 |

From Table 1, it is understood that addition of a fragrance ingredient containing cis-3-hexenol makes it possible to markedly mask the ammonia odor of ammonia containing compositions and hence provides a preferred fragrance.

The disclosure of Japanese priority Application No. 2000-286644, filed September 21, 2000 is hereby incorporated by reference into the present application.

Obviously, numerous modifications and variations of the present invention are possible in light of the above teachings. It is therefore to be understood that within the scope of the appended claims, the invention may be practiced otherwise than as specifically described herein.

## Claims

1. A hair cosmetic formulation, comprising:
(A) a fragrance ingredient comprising cis-3-hexenol; and
(B) at least one ingredient selected from the group consisting of ammonia, monoethanolamine, and an aromatic alcohol penetration promoter.

2. The hair cosmetic formulation according to Claim 1, wherein the content of cis-3-hexenol in the formulation ranges from 0.1 to 50 wt.% of the fragrance ingredient (A).

3. The hair cosmetic formulation according to Claim 1 or 2, wherein said fragrance ingredient (A) further comprises at least one substance selected from the group consisting of cis-3-hexenol esters, trans-2-hexenol, alcohol C-6, dimethol, dihydromyrcenol, citronellol, geraniol, linalool, Magnol™, 2-methyl-5-(2,2,3-trimethyl-3-cyclopenten-1-yl)-4-penten-2-ol, eugenol, p-cresol, 3,3-dimethylcyclohexyl methyl ketone, acetyl diisoamylene, -methyl ionone, 1-menthone, cis-3-hexenyl methyl carbonate, ethyl 2-methylpentanoate, ethyl tricylo[5.2.1.0.^{2,6}]decan-2-ylcarboxylate, o-t-butylcyclohexyl acetate, p-cresyl acetate, 1,8-cineole, Anethole™, estragol, rose oxide and limonene.

4. The hair cosmetic formulation according to any one of the Claims 1 to 3, which is an oxidation hair coloring formulation or a hair bleaching formulation.

5. The hair cosmetic formulation according to any one of the Claims 1 to 4, wherein the content of ingredient (A) ranges from 0.1 to 1.0%, especially from 0.3 to 0.8%, based on the weight of the composition.

6. The hair cosmetic formulation according to Claim 5, wherein the content of ingredient (A) ranges from 0.3 to 0.8 wt.%, based on the weight of the composition.

7. The hair cosmetic formulation according to any one of the Claims 1 to 6, wherein the content of ammonia in the composition ranges from 0 to 3% by weight.

8. The hair cosmetic formulation according to Claim 7, wherein the content of ammonia in the composition ranges from 0 to 1% by weight.

9. The hair cosmetic formulation according to any one of the Claims 1 to 8, wherein the aromatic alcohol solvent has formula (1): 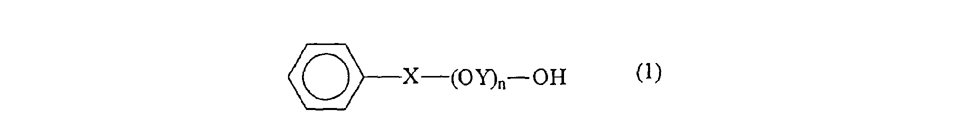 wherein n stands for 0 or 1 with the proviso that when n = 0, X is a linear or branched alkylene, alkenylene or alkylenoxy group having 1 to 6 carbon atoms and that when X is alkyleneoxy, the oxygen atom of the alkylenoxy group is bonded to the benzene ring, and, when n=1, X and Y each independently represent a linear or branched alkylene group having 1 to 6 carbon atoms.

10. The hair cosmetic formulation according to Claim 9, wherein the aromatic alcohol solvent is benzyl alcohol, phenylethyl alcohol, phenoxyethanol, phenoxyisopropanol, -methylbenzyl alcohol, , -dimethylbenzyl alcohol, -propylbenzyl alcohol, 2-benzyloxyethanol and 3-benzyloxybutanol.

11. The hair cosmetic formulation according to any one of the claims 1 to 10, wherein the aromatic alcohol, as a penetration promoter, is present in the composition in an amount ranging from 0 to 40 wt.%.

12. The hair cosmetic formulation according to Claim 11, wherein the aromatic alcohol, as a penetration promoter, is present in the composition in an amount ranging from 5 to 25 wt.% in the cosmetic hair composition.

13. The hair cosmetic formulation according to any one of the Claims 1 to 12, wherein, when the formulation is an oxidation coloring composition or an oxidation dye intermediate, the formulation additionally comprises a color-developing substance and a coupling agent.

14. The hair cosmetic formulation according to Claim 13, wherein the color-developing substance is a p-phenylenediamines, 2,5-diaminopyridines, p-aminophenols, o-aminophenols, o-phenylenediamines or 4,5-aminopyrazoles.

15. The hair cosmetic formulation according to Claim 13 or 14, wherein the coupling agent is a m-phenylenediamine, a m-aminophenol, a m-hydroxybenzene, a hydroxyindole, a naphthol or a phenol.

16. The hair cosmetic formulation according to any one of the Claims 1 to 15, wherein the formulation comprises at least one additive selected from the group consisting of viscosity/gel strength modifiers, oils and fats, waxes, hydrocarbons, polyhydric alcohols, amides, silicone derivatives, cationic surfactants, anionic surfactants, amphoteric surfactants, nonionic surfactants, nonionic high-molecular substances, cationic high-molecular substances, anionic high-molecular substances, amphoteric high-molecular substances, protein derivatives and amino acids, preservatives, chelating agents, stabilizers, oxidation inhibitors, plant extracts, crude drug extracts, vitamins, color additives, fragrances, pigments and ultraviolet absorbers.

17. Use of the hair cosmetic formulation as defined in any one of the Claims 1 to 16 as a hair coloring formulation.
